# EUROPÄISCHE PATENTANMELDUNG

(11) **EP 3 072 466 A1**
(43) Veröffentlichungstag der Anmeldung: **28.09.2016**
(21) Anmeldenummer: 15161026.8
(22) Anmeldetag: 26.03.2015
(51) Int. Cl.: A61B 17/88

(54) **Stabtrennzange**

(71) Anmelder: Silony Medical International AG, 8500 Frauenfeld (CH)
(72) Erfinder: Heuer, Frank, 70794 Filderstadt (DE); Trautwein, Frank, 70794 Filderstadt (DE)
(74) Vertreter: Stolmár & Partner Patentanwälte PartG mbB

(57) **Zusammenfassung**

Die Erfindung betrifft eine Stabtrennzange (10) zum Trennen eines Verbindungsstabs für Wirbelimplantate, umfassend ein erstes Trennelement (12) mit zumindest einer Aufnahme (12a) für den Verbindungsstab und einer Schneidkante (12b) an der Aufnahme; ein zweites Trennelement (14) mit zumindest einer Aufnahme (14a) für den Verbindungsstab und einer Schneidkante (14b) an der Aufnahme, wobei das erste und zweite Trennelement (12,14) derart miteinander verbunden sind, dass sie relativ zueinander bewegbar sind, einen Hebelmechanismus (16), der zumindest zwei miteinander verbundene Schenkel (13, 15) aufweist und mit mindestens einem Betätigungsgriff (22, 23) versehen ist, wobei der Hebelmechanismus (16) mit dem ersten und zweiten Trennelement (12,14) verbunden ist und bei der Betätigung diese relativ zueinander bewegt, so dass ein Trennschnitt ausgeführt wird, und wobei der Hebelmechanismus (16) zusammen mit den Trennelementen (12,14) eine sich derart verändernde Kraftübersetzung aufweist, dass bei der Betätigung des Hebelmechanismus (16) die Schneidkraft der Trennelemente (12, 24) ansteigt, wobei die Schneidposition der Schneidkanten (12b, 14b) der Trennelemente (12,14) erst erreicht ist, wenn zumindest 50% des Hebelwegs des Hebelmechanismus (16) zurückgelegt sind.

## Beschreibung

### Gebiet der Erfindung

Die Erfindung betrifft eine Stabtrennzange zum Abtrennen von bei chirurgischen Eingriffen verwendeten Stäben, insbesondere aus Titan, Titanlegierungen, CoCr oder Edelstahl.

### Stand der Technik

Zur Stabilisierung der Wirbelsäule, beispielweise bei einem Bandscheibenvorfall, werden Wirbelsäulenstützelemente bzw. Verankerungselemente wie mono- oder polyaxiale Pedikelschrauben verwendet. Die Pedikelschrauben werden an den vorgesehenen Stellen in dem Wirbel der Wirbelsäule verankert und über einen Verbindungsstab miteinander verbunden. Der Verbindungsstab ist dabei je nach Anforderung schon vorgebogen oder wird durch den Anwender konturiert, da die Verankerungselemente nicht nur zur Stabilisierung von zwei Wirbeln dienen können, sondern auch, um Schiefstellungen des Rückgrats zu korrigieren. Dazu wird der Verbindungsstab in die jeweiligen Verankerungselemente eingesetzt, wobei seine Krümmung in etwa der Krümmung der Wirbelsäule entspricht, um das Einsetzen des Verbindungsstabs einfach zu gestalten. Nach der gewünschten Positionierung des Verbindungsstabs werden die jeweiligen Schrauben in den Verankerungselementen festgedreht, so dass der Verbindungsstab in seiner gewünschten Position mit den Verankerungselementen stabil befestigt ist. Danach werden dann die Enden des Verbindungsstabs, die aus den jeweiligen Verankerungselementen überstehen, mit einer speziellen Zange abgetrennt. Bei herkömmlichen Zangen zum Abtrennen von solchen oder ähnlichen Verbindungsstäben verwenden häufig zur Vergrößerung der auf den Verbindungsstab wirkenden Kraft eine Zange mit einem Hebelmechanismus, die auf die Handgriffe aufgebrachte Kraft verstärkt und so an den Schneidelementen, die auf den Verbindungsstab zum Abscheren aufgebracht verstärkt.

Problematisch ist jedoch, dass die Zangen aus dem Stand der Technik ziemlich groß dimensioniert werden müssen, da auch bei einem Kniehebel die auf den Handgriffe aufzubringende Kraft sehr hoch ist. Denn gerade zu Beginn des Schneidprozesses entfaltet die Kniehebelverstärkung noch nicht ihre volle Wirkung und die aufzubringenden Kräfte zum Trennen des Verbdingungsstabs sind daher recht groß, weswegen die Zangen daher auch sehr stabil und massiv ausgeführt werden müssen.

Um den zu Beginn der Schneidarbeit erhöhten Kraftaufwand zu kompensieren, wurde bisher einfach der Hebelarm an den Griffen verlängert. Eine solche Verlängerung ist jedoch durch die jeweiligen Umstände konstruktiv begrenzt, das heißt die Hebel können nicht beliebig lang gemacht werden, da der Anwender der Zange zum Einen üblicherweise nah am Patienten sein sollte und vor allem machen längere Hebelarme die Zange unhandlicher, so dass das Einsetzen des Verbindungsstabs in die Aufnahme weniger präzise und damit schwieriger ist. Außerdem bekommen die Trennzangen durch längere Hebelarme noch mehr Gewicht und passen schlecht in die Lagerungssiebe.

### Darstellung der Erfindung

Aufgabe der Erfindung ist es daher, eine Stabtrennzange insbesondere für Stäbe aus Titan, Titanlegierungen, CoCr oder Edelstahl, bereitzustellen, bei der das Gewicht der Stabtrennzange reduziert werden kann, um eine präzisere Handhabung zu ermöglichen.

Dieses Problem wird gelöst durch eine Stabtrennzange nach Anspruch 1. Weitere die Erfindung ausgestaltende Merkmale sind in den Unteransprüchen enthalten.

Eine erfindungsgemäße Stabtrennzange zum Trennen eines Verbindungsstabs für Wirbelimplantate umfasst ein erstes Trennelement mit zumindest einer Aufnahme für den Verbindungsstab und einer Schneidkante an der Aufnahme, ein zweites Trennelement mit zumindest einer Aufnahme für den Verbindungsstab und einer Schneidkante an der Aufnahme, wobei das erste und zweite Trennelement derart miteinander verbunden sind, dass sie relativ zueinander insbesondere rotatorisch bewegbar sind, und einen Hebelmechanismus, der zwei miteinander verbundene Schenkel aufweist und mit zwei Betätigungsgriffen versehen ist, wobei der Hebelmechanismus mit dem ersten und zweiten Trennelement verbunden ist und bei der Betätigung diese relativ zueinander bewegt, so dass ein Trennschnitt ausgeführt wird, und wobei der Hebelmechanismus zusammen mit den Trennelementen eine sich derart verändernde Kraftübersetzung aufweist, dass bei der Betätigung des Hebelmechanismus die Schneidkraft der Trennelemente ansteigt, wobei die Schneidposition der Schneidkanten der Trennelemente erst erreicht ist, wenn zumindest 50% des Hebelwegs des Hebelmechanismus zurückgelegt sind. Vorzugsweise sollen zumindest 60%, 70%, 80% oder zumindest 90% des Hebelwegs, insbesondere 95 % des Hebelwegs zurückgelegt sein, bevor die Schneidkanten mit dem Verbindungsstab in Kontakt treten. Dadurch wird gewährleistet, dass die Schneidkanten erst dann angreifen, wenn der sich verstärkende Hebelmechanismus so weit bewegt hat, dass die Kraftübersetzung wesentlich größer ist, als bei herkömmlichen Zangen. Durch die bessere Hebelübersetzung kann die Zange kleiner dimensioniert werden, da die auf die Handgriffe aufzubringende Kraft kleiner sein kann.

Die Schneidposition der Schneidkanten kann beispielsweise festgelegt werden, indem die Aufnahme für den Verbindungsstab an einem Punkt in den Trennelementen konstruiert wird, an dem der Hebelweg schon den geforderten Anteil zurückgelegt wurde, so dass die notwendige Änderung der Übersetzung schon erreicht ist. Dann können die Schneidkanten unmittelbar nach dem Einlegen des Verbindungsstabs an diesen angreifen und den Stab trennen. Eine andere Möglichkeit ist, zumindest eine Aufnahme an einem Trennelement größer auszuführen, so dass der Verbindungsstab in die Aussparung der Aufnahme eingesetzt werden kann, bei Betätigung des Hebelmechanismus die Schneidkante jedoch erst an den Verbindungsstab herangeführt wird und erst dann den Verbindungsstab berührt, wenn der erforderliche Hebelweg zurückgelegt ist. Der Hebelweg des Hebelmechanismus ist daher nicht durch die konstruktive Ausgestaltung der Zange definiert, sondern wird definiert über den Weg, den die Hebel von der theoretisch geringsten Kraftübersetzung bis zur höchsten Kraftübersetzung durchlaufen müssen. Dies wird nachfolgend auch noch für den Kniehebel definiert. Die Verbindungen der Stabtrennzange sind vorzugsweise einfach zu öffnen und zu schließen, damit die Zange leicht gereinigt werden kann.

Ferner ist zumindest ein Trennelement der Stabtrennzange abgeknickt ausgebildet. Insbesondere in einem Winkel von zumindest 100°, weiter vorzugsweise zumindest 130° und weiter vorzugsweise 160°. Mögliche Obergrenzen könnten 175°, 170° oder 165° sein. Eine solche Ausgestaltung ermöglicht eine schmale Ausgestaltung am distalen Ende der Stabtrennzange. Eine solche schmale Konstruktion ist vorteilhaft, da im Bereich des Verbindungsstabs am offenen Gewebe operiert wird und die Verletzungsgefahr für den Patienten verringert wird.

Der Hebelmechanismus der Stabtrennzange umfasst vorzugsweise zumindest einen Kniehebel, vorzugsweise jedoch zwei symmetrisch angeordnete. Der Kniehebel ist eine konstruktiv einfache Lösung eines solchen Hebelmechanismus, die bei der Verwendung eine größere Kraftübersetzung ermöglicht.

Die Stabtrennzange kann ferner am Hebelmechanismus eine Schnittstelle für das Befestigen von Handhebeln und/oder von Hebelverlängerungen aufweisen. Mit einer solchen Schnittstelle können in einfacher Weise verschiedene Handhebel verwendet werden und so nach Bedarf an die Bedingungen für die Stabtrennzange angepasst werden.

Ferner kann vorzugsweise zumindest eine Aufnahme des ersten und zweiten Trennelements eine länglich ausgebildete Aussparung aufweisen, die entlang der Bewegungslinie des eingespannten Verbindungsstabs verläuft und entlang der Bewegungslinie größer als der Durchmesser des Verbindungsstabs ist. Das heißt eine Aufnahme kann bspw. dem Durchmesser des Verbindungsstabs entsprechen, so dass er stabil und fest gehalten werden kann, die andere Aufnahme ist als längliche Aussparung ausgebildet, so dass die Schneidkante erst mit dem Stab in Kontakt gerät, wenn die Kraftübersetzung des Hebelmechanismus ausreichend gestiegen ist. "Länglich" bzw. "lang" bedeutet hierbei lediglich, dass die Aussparung in der Bewegungsrichtung größer als der Durchmesser ausgebildet ist. Die Größendifferenz kann also von einem halben Millimeter bis zu einem Zentimeter oder mehr betragen, wobei ihre Ausmaße von der Konstruktion der Zange abhängen. Bei einer solchen Ausführungsform kann sichergestellt werden, dass die Schneidkanten erst bei einer angemessenen Kraftübersetzung in Kontakt mit dem Verbindungsstab gelangen, und gleichzeitig die übliche Handhabung von Stabtrennzangen nicht verändert wird. In einer weiteren Ausgestaltung ist in beiden Trennelementen eine solche Aussparung vorgesehen. Dadurch können die jeweiligen Aussparungen kürzer ausgebildet werden. Die Aussparungen sind insbesondere gegenläufig und weiter vorzugsweise zum Verbindungspunkt der beiden Trennelemente gerichtet, so dass sich der Verbindungsstab nach innen bewegt. Ferner können die Trennelemente an der Aussparung Vorsprünge aufweisen, so dass die Aussparung hakenförmig ausgebildet ist. Sobald sich die Trennelemente bewegt haben, verhindern die Vorsprünge so, dass der Verbindungsstab sich unbeabsichtigt aus der Aussparung lösen kann.

Weiterhin ist bevorzugt, dass die Aussparungen der Trennelemente bei betätigtem Hebelmechanismus in der Endstellung nicht vollständig abgedeckt sind, insbesondere zu höchstens 70%, vorzugsweise zu höchstens 50% und weiter vorzugsweise zu höchstens 30%. Dadurch, dass in der Endstellung die beiden Aussparungen nicht komplett abgedeckt werden müssen, kann auch die Endstellung der Trennelemente zueinander einfacher eingestellt werden. Denn die Endstellung muss nun nicht zwingend eine vollständige Überdeckung erreichen.

An den Trennelementen kann eine Mehrzahl von zueinander gehörenden Aufnahmen mit jeweils vorhandenen Schneidkanten vorgesehen sein, die bevorzugt unterschiedliche Ausmaße aufweisen. Dadurch können verschiedene Stabdurchmesser mit einer Zange bearbeitet werden, gleichzeitig die Verbindungsstäbe aber stabil in den dafür entsprechenden Aufnahmen gehalten und abgetrennt werden.

Ferner ist bei der Stabtrennzange vorzugsweise die Schneidposition der Schneidkanten der Trennelemente erst dann erreicht, wenn die miteinander verbundenen Schenkel des Hebelmechanismus, die mit den Trennelementen verbunden sind, zueinander in einem Winkel von 0° bis 45° Grad oder 135° bis 180° Grad stehen. Diese Ausgestaltung gilt für die Verwendung eines Kniehebels und kann weiter durch die oben genannte prozentualen Einschränkungen des Hebelwegs begrenzt werden (z.B. entsprechen 90% des Hebelwegs 0° bis 4,5° Grad bzw. 175,5° bis 180° Grad).

### Kurze Beschreibung der Figuren

Fig. 1 zeigt eine isometrische Ansicht einer erfindungsgemäßen Stabtrennzange;
Fig. 2a zeigt die Stabtrennzange aus Fig. 1 in einer geöffneten Position und als schematisches Schaubild;
Fig. 2b zeigt eine vergrößerte Aufnahme der Trennelemente der Stabtrennzange aus Fig. 2a;

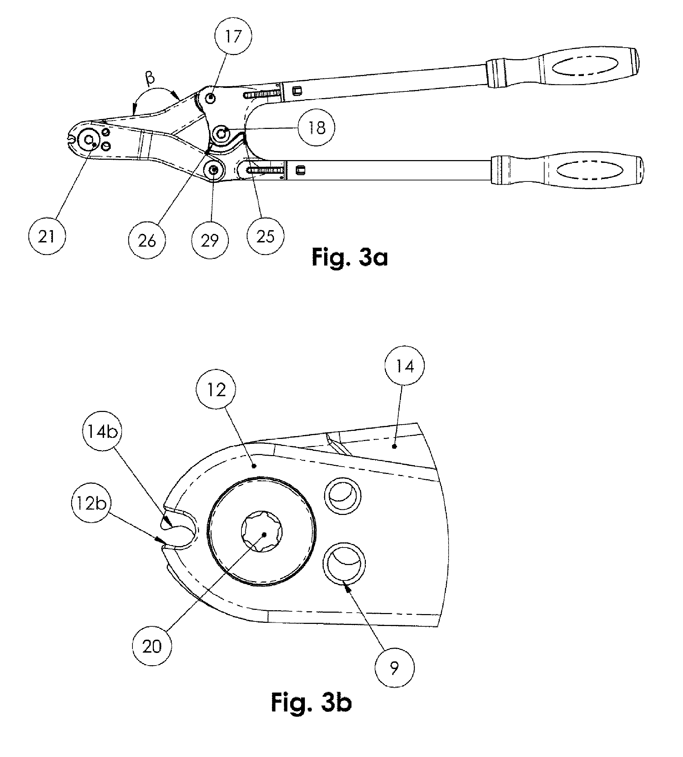

Fig. 3a zeigt die Stabtrennzange aus Fig. 1 in einer geschlossenen Position und als schematisches Schaubild;
Fig. 3b zeigt eine vergrößerte Aufnahme der Trennelemente der Stabtrennzange aus Fig. 3b;
Fig. 4 zeigt eine Schnittstelle des Hebelmechanismus für einen Handhebel;
Fig. 5 zeigt die Schnittstelle aus Fig. 4, an der ein Handhebel befestigt ist;
Fig. 6 ist ein schematisches Diagramm, dass eine Beziehung zwischen dem Winkel der Hebelarme zueinander und den an den Griffen und den Schneidkanten wirkenden Kräften zeigt; und
Fig. 7a-c zeigen eine schematische Darstellung der theoretischen Hebelstellungen der Stabtrennzange aus Figur 1.

### Bevorzugte Ausführungsformen der Erfindung

Im Folgenden werden die Begriffe "distal" und "proximal" verwendet. "Distal" bedeutet dabei die Richtung zum Patienten hin, während "proximal" eine Richtung bezeichnet, die vom Patienten weg zeigt.

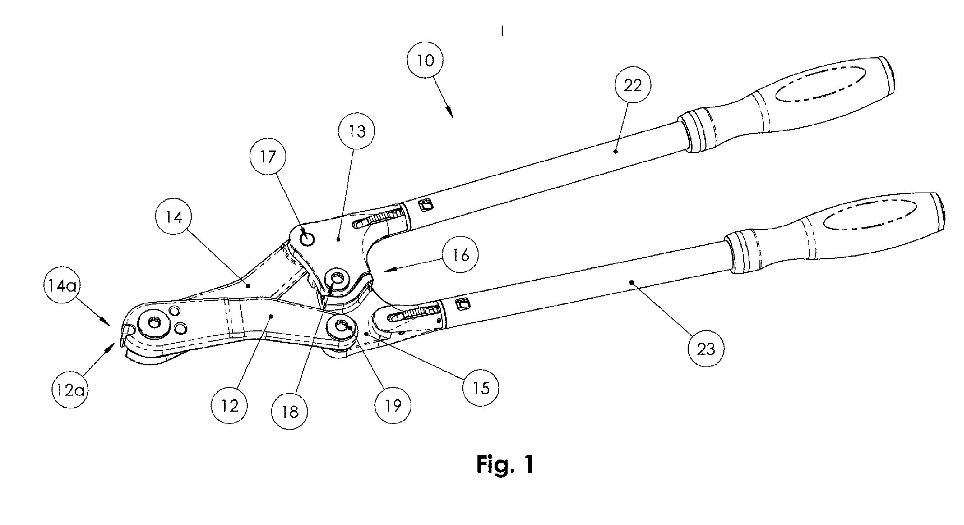

Eine bevorzugte Ausführungsform der Erfindung ist in Fig. 1 gezeigt. Die Stabtrennzange besteht aus einem Hebelmechanismus 16, der mit den Trennelementen 12 und 14 verbunden ist. Vorzugsweise bilden die Trennelemente 12, 14 und der Hebelmechanismus 16 zumindest einen Kniehebel aus, insbesondere einen doppelten und vorzugsweise symmetrischen Kniehebel, wie er in der Stabtrennzange 10 vorgesehen ist, die in den Figuren gezeigt ist.

Der Kniehebel ändert oder genauer gesagt vergrößert mit jedem Betätigungsmillimeter sein Übersetzungsverhältnis. Dadurch entsteht im gebeugten Zustand eine Hohlweg-Übersetzung bei einer geringen Kraftübersetzung. Je mehr der Kniehebel den durchgestreckten Zustand erreicht, desto mehr nimmt bei gleichbleibender Betätigungswinkeländerung (bzw. Betätigungswegänderung) der Hubweg ab, die Presskraft dagegen zu.

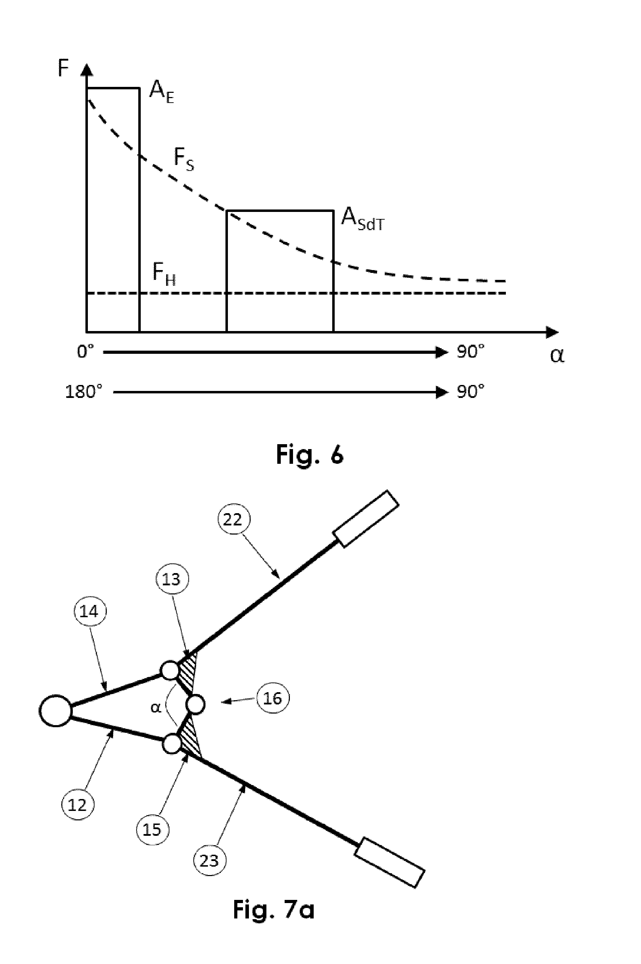

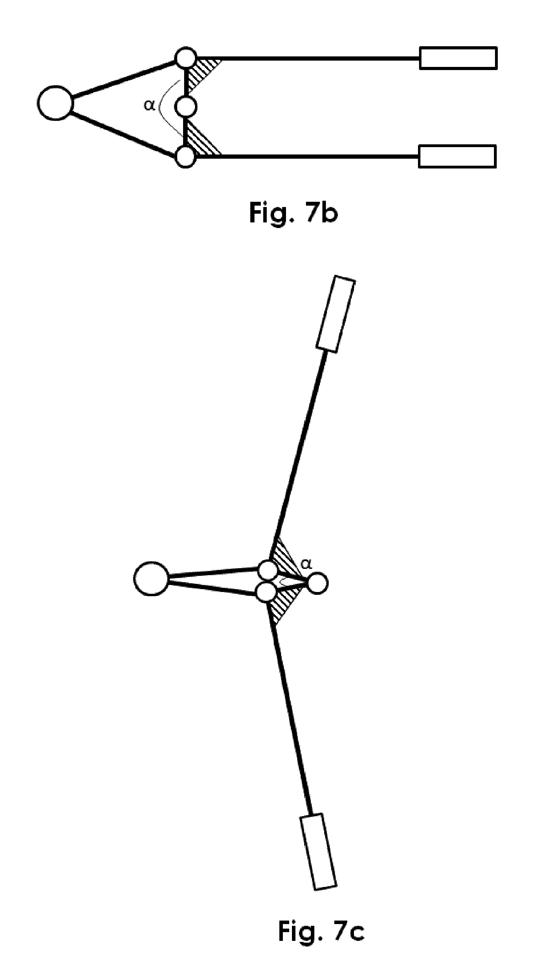

Vorliegend sind zwei Trennelemente 12 und 14 über eine Verbindung 18 rotatorisch miteinander verbunden. Der Hebelmechanismus 16 besteht in der bevorzugten gezeigten Ausführungsform aus zwei miteinander über ein Gelenk rotatorisch verbundenen Hebelelementen 13,15, die jeweils mit einem Trennelement 12, 14 über ein Gelenk 17, 19 rotatorisch verbunden sind. Diese Ausgestaltung entspricht einem Kniehebel, der eine sich verändernde Kraftübersetzung aufweist. Je mehr die Gelenkpunkte 17,18, 19 in eine Flucht gelangen (dass also alle drei Gelenkpunkte auf einer Geraden liegen), desto größer wird die Übersetzung der Kraft. Für den Kniehebel bedeutet dass, dass die Übersetzung am geringsten ist, wenn die Hebelelemente in einem Winkel α von 90° Grad zueinander stehen (siehe Fig 7a). Wenn die Hebelelemente nun den Winkel auf bis zu 180° Grad vergrößern (siehe Fig. 7b) oder ihn entsprechend auf 0° Grad verkleinern (siehe Fig. 7c), steigt die Übersetzung an, so dass am Ende die an den Schneidkanten wirkende Kraft exponentiell steigt.

Die Gelenkverbindungen 17,18, 19, 21 sind vorzugsweise einfach zu öffnen, so dass die einzelnen Elemente voneinander getrennt und einfacher gereinigt werden können. Beispielsweise sind für die Gelenke Schraubverbindungen, eventuell mittels Schrauben oder Gewindebolzen, eine einfache und effektive Befestigungsmöglichkeit.

Jedes Trennelement 14, 12 weist zumindest eine Aussparung 12a, 14a auf, die eine Aufnahme ausbildet, in die der Stab eingelegt werden kann. Diese Aussparung 12a, 14a ist vorzugsweise am distalen Ende des Trennelements 14, 12 vorgesehen, kann aber auch als Durchgangsbohrung 9 vorgesehen sein. Es ist möglich in einem Trennelement lediglich eine Aussparung 12a, 14a vorzusehen, bevorzugt sind jedoch zwei, drei oder mehrere Aussparungen, beispielsweise für Stäbe mit verschiedenen Durchmessern. Die Aussparung ist dabei in der gezeigten Ausführungsform vorzugsweise relativ nah am Gelenkpunkt 20 angeordnet (bspw. innerhalb von 50 mm), da dadurch eine bessere Hebelwirkung erzeugt wird. Wenn mehr als eine Aussparung 9, 12a, 14a in einem Trennelement 12, 14 vorgesehen ist, dann werden diese umfänglich um den Gelenkpunkt 20 in vorzugsweise gleichem Abstand zum Gelenkpunkt 20 und/oder auch in gleichem Abstand in Umfangsrichtung angeordnet. Das Befestigungsmittel, das die beiden Trennelemente miteinander insbesondere rotatorisch verbindet überdeckt die Aussparungen jedoch nicht. Es gelten für die in Durchgangsbohrungen 9 die gleichen Ausgestaltungen, die nachfolgend für die Aussparungen erläutert werden, sofern nichts anderes erwähnt wird. Ferner sind die Durchgangsbohrungen 9 ebenfalls in beiden Trennelementen vorgesehen. Die Durchgangsbohrungen können unterschiedliche Radien zum Drehzentrum für unterschiedliche Verbindungsstabdurchmesser aufweisen, um eine gleiche oder zumindest ähnliche später erläuterte prozentuale Verdeckung Schneidkanten bei gleichem Betätigungsweg der Hebelarme zu erzielen.

Unter Bezugnahme auf das Diagramm in Fig. 6 wird nun der Unterschied zu den herkömmlichen Stabtrennzangen erläutert. Üblicherweise sind die Aussparungen für die Aufnahme in einer Stabtrennzange so angeordnet, dass der Verbindungsstab bei komplett geöffneter Hebelstellung der Stabtrennzange eingelegt werden kann und der Anwender beginnt, die Hebelarme der Stabtrennzange zusammenzudrücken. Unmittelbar nach Betätigung der Hebelarme der Zange geraten die Schneidkanten an den Verbindungsstab und erzeugen Scherkräfte, die auf den Verbindungsstab wirken. Dabei ist die offene Stellung der Stabtrennzange so ausgestaltet, dass die Kraftübersetzung im niedrigen Bereich liegt, weswegen viel Kraft zum Schneiden des Verbindungsstabs benötigt wird. Dieser Bereich ist in Fig. 6 mit A_{SdT} bezeichnet. Bei der vorliegenden Stabtrennzange 10 ist die Aussparung 12a, 14a, 9 der Trennelemente 12, 14 oder die Platzierung der Aussparung 12a, 14a, 9 derart ausgestaltet, dass die Schneidkanten 12b, 14b erst in einem Bereich an den Verbindungsstab angreifen, der in dem Diagramm aus Fig. 6 mit A_{E} bezeichnet ist. Wenn an den Hebelarmen 22 und 23 eine konstante Kraft anliegt, steigt bei dem vorliegenden Hebelmechanismus 16 mit sich verändernder Übersetzung die Kraft F_{S} zwischen den Schneidkanten bei gleichbleibender Hebelkraft F_{H} an den Hebelarmen stetig an. Die Trennelemente 12, 14 bewegen sich entsprechend zueinander, im vorliegenden Fall rotatorisch um den Gelenkpunkt 18. Bei einer erfindungsgemäßen Ausführungsform ist die Aufnahme 12a, 14a, 9 so ausgestaltet oder vorgesehen, dass die Schneidkanten 12b, 14b erst dann in die Schneidposition gelangen (also gleichzeitig mit dem Verbindungsstab in Kontakt geraten), wenn der Hebelweg, den Hebelmechanismus 16 zurücklegt, schon wesentlich größer ist und die Übersetzung sich stark verbessert hat. Dies ist im Diagramm in Fig. 6 in dem Bereich A_{E} der Fall. Dadurch wird die Kraft, die auf die Hebelarme aufgebracht werden muss, im Vergleich zum Stand der Technik extrem verringert.

Vorzugsweise sollen die Schneidkanten 12b, 14b erst dann eingreifen, wenn zumindest 50 % des Hebelwegs des Hebelmechanismus 16 zurückgelegt sind. Vorzugsweise sollen zumindest 60%, 70%, 80% oder zumindest 90% des Hebelwegs, insbesondere 95 % des Hebelwegs zurückgelegt sein, bevor die Schneidkanten 12b, 14b mit dem Verbindungsstab in Kontakt treten.

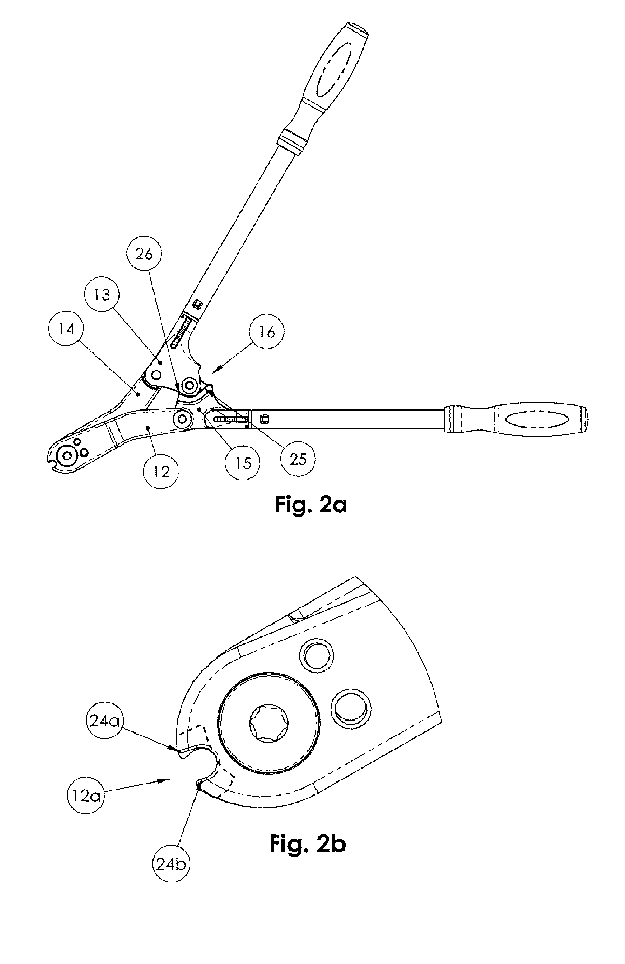

Um die Position der Schneidkanten 12b, 14b erst später an den Stab angreifen zu lassen (später im Sinn von "bei erhöhter Kraftübersetzung"), gibt es prinzipiell mehrere Möglichkeiten. Eine ist es, zwei Aussparungen in den Trennelementen 12, 14 vorzusehen, wie sie derzeit üblicherweise verwendet werden und die sofort nach Betätigung der Hebelarme 22, 23 an dem Verbindungsstab anliegen, dabei aber darauf zu achten, dass die Hebelarme 13, 15 des Hebelmechanismus 16 sich schon in einem Bereich A_{E} befinden, der die erhöhte Kraftübersetzung bereitstellt. Dies kann konstruktiv beispielsweise durch eine Wegbeschränkung wie eine Kette oder einen Bügel, die zwischen den Hebelarmen 22, 23 vorgesehen sind, gelöst werden, die die Bewegungsfreiheit der Hebelarme 22, 23 beschränken. Alternativ kann die Bewegungsfreiheit der Hebelarme 22, 23 auch durch entsprechende Anschläge 25, 26 begrenzt werden, die die offenen und geschlossenen Positionen der Hebelarme 22, 23 definieren. Das führt jedoch dazu, dass der Hebelweg, den die Hebelarme 22, 23 gehen, relativ klein ist, was die Anwender möglicherweise nicht gewohnt sind. Um dem Anwender das Gefühl zu geben, dass sich die Anwendung der Stabtrennzange nicht großartig geändert hat und der Hebelweg daher dem des Standes der Technik entspricht, kann eine Aussparung auch größer ausgeführt werden, das heißt, dass zumindest eine Aufnahme als längliche Ausnehmung ausgebildet ist, so dass die Schneidkante erst dann mit dem Verbindungsstab in Kontakt gerät, wenn schon der Großteil des Hebelwegs des Hebelmechanismus zurückgelegt ist. Eine solche Ausführungsform kann man in Fig. 2b sehen, in der eine mögliche längliche Aussparung eines Trennelements durch die gestrichelte Linie schematisch angedeutet ist. In einer weiteren bevorzugten und ebenfalls in den Figuren dargestellten Ausführungsform ist die längliche Aussparung radial nach innen ausgebildet, insbesondere spiralförmig. Dabei ist die spiralförmige nach innen laufende Aussparung vorzugsweise in beiden Trennelementen vorgesehen und der spiralverlauf an den beiden Trennelementen gegenläufig. Dadurch rutscht der Verbindungsstab bei Betätigung der Stabtrennzange 10 weiter nach innen und verbessert so die Hebelwirkung für die Schneidkanten, da der Verbindungsstab näher am Gelenkpunkt 20 liegt. Dadurch können die Schneidkanten erst ausreichend spät in den Hebelweg mit dem Verbindungsstab eingreifen. Wie schon erwähnt muss die längliche Form nicht mit bloßem Auge erkennbar sein. Denn bei geeigneter konstruktiver Ausgestaltung kann der Weg, den der Verbindungsstab in der Aussparung zurücklegt, auch nur 500 Mikrometer oder sogar weniger sein. Um die längliche Aussparung kleiner ausbilden zu können, kann diese in beide Trennelemente ausgebildet sein, so dass die Bewegungslinie, die der Verbindungsstab zurücklegt, auf beide Trennelemente verteilt werden kann. Beispielhaft sei hier auf die gestrichelt dargestellte Aussparung in Figur 2b verwiesen, die entweder doppelt so groß in einem Trennelement oder jeweils halb so groß in beiden Trennelementen 12, 14 ausgebildet sein kann. Die Durchgangsbohrungen 9 können diese längliche Ausgestaltung selbstverständlich auch aufweisen.

Vorzugsweise weist zumindest eine Aussparung zumindest eines Trennelements zumindest einen hakenförmigen Schnabel 24a an der Aussparung 12a auf, so dass bei Betätigung Stabtrennzange 10 der Verbindungsstab in der Aussparung gehalten werden kann. Bevorzugt ist aber, dass an beiden Aussparungen 12a, 14a zumindest ein solcher Schnabel 24a vorgesehen ist und/oder dass ein Trennelement 12, 14 einen oberen Schnabel 24a und einen unteren Schnabel 24b jeweils am oberen und unteren Rand der Aussparung 12a, 14a aufweisen. Solche Haken können natürlich nicht an den in sich geschlossenen Durchgangsbohrungen 9 vorgesehen sein.

Die Trennelemente 12, 14 sind vorzugsweise in Längsrichtung (also vom proximalen Ende zum distalen Ende) abgeknickt ausgebildet. Dadurch wird ermöglicht, dass die Stabtrennzange 10 sehr schlank ausgebildet sein kann. Der Knickwinkel β der Trennelemente sollten vorzugsweise zumindest 100°, weiter vorzugsweise zumindest 130° und insbesondere zumindest 160° sein.

In der Endstellung, also der geschlossenen Stellung in der Stabtrennzange 10 werden die Aussparungen 12a, 14a der Trennelemente 12, 14 vorzugsweise nicht komplett vom jeweils anderen Trennelement abgedeckt. Es ist bevorzugt, dass das jeweils andere Trennelement 12, 14 die Aussparung zu höchstens 70% abdeckt, vorzugsweise zu höchstens 50% und weiter vorzugsweise zu höchstens 30%. Das heißt, dass lediglich höchstens 70%, bzw. höchstens 50% oder höchstens 30% der Schnittkante in die andere Aussparung hineinragt. Bei einer solchen Schnittspaltreduzierung werden insbesondere die gebräuchlichsten Verbindungsstäbe aus Kobalt-Chrom, Titan, Titanlegierungen und Edelstahl bereits getrennt. Dies ermöglicht eine größere konstruktive Freiheit, da nicht mehr dafür Sorge getragen werden muss, dass in der geschlossenen Position der Stabtrennzange die Schnittkanten die Aufnahmen vollkommen überdecken.

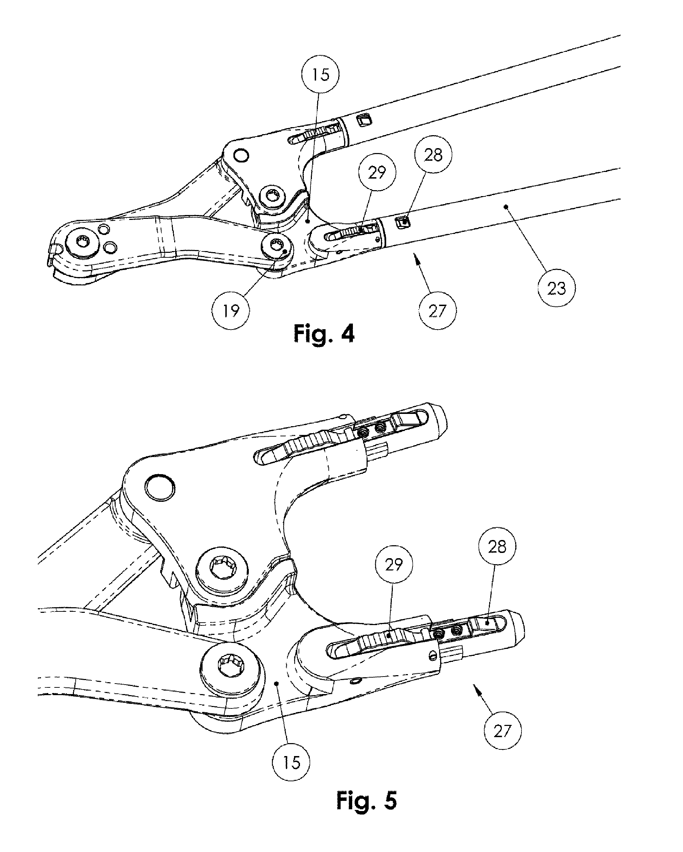

Die Hebelarme 22, 23 zum Betätigen der Stabtrennzange 10 können einteilig in den Hebelmechanismus 16 integriert sein. Bevorzugt ist jedoch, dass der Hebelmechanismus 16 Schnittstellen 27 aufweist, die in Fig. 4 und 5 gezeigt sind. Mit diesen Schnittstellen 27 können einfach verschiedene Hebelarme 22, 23 angebracht werden, indem sie einfach an den Hebelmechanismus angesteckt werden. Gleichzeitig ist auch eine vereinfachte Reinigung des Hebelmechanismus der Stabtrennzange möglich. Im vorliegenden Fall ist die Schnittstelle 27 durch ein Klicksystem ausgebildet, das einen Eingriffshaken 28 aufweist, auf den der Hebelarm 23 aufgeschoben wird und in den der Haken 28 dann eingreift. Der 28 Haken ist dazu federnd gelagert und kann ebenfalls in Axialrichtung eines Hebelarms mittels des Betätigungsknopfes 29 verschoben werden, so dass er sich unter bzw. in den Hebelarm 23 schieben lässt, der Hebelarm dann verdreht werden kann und von der Schnittstelle genommen werden kann. Andere Möglichkeiten einer Schnittstelle sind beispielsweise Gewinde, auf mittels denen die Hebelarme einfach auf die Schnittstelle aufgeschraubt oder in diese eingeschraubt werden können.

## Patentansprüche

1. Stabtrennzange (10) zum Trennen eines Verbindungsstabs für Wirbelimplantate, umfassend
ein erstes Trennelement (12) mit zumindest einer Aufnahme (12a) für den Verbindungsstab und einer Schneidkante (12b) an der Aufnahme;
ein zweites Trennelement (14) mit zumindest einer Aufnahme (14a) für den Verbindungsstab und einer Schneidkante (14b) an der Aufnahme, wobei das erste und zweite Trennelement (12,14) derart miteinander verbunden sind, dass sie relativ zueinander bewegbar sind;
einen Hebelmechanismus (16), der zumindest zwei miteinander verbundene Schenkel (13,15) aufweist und mit mindestens einem Betätigungsgriff (22, 23) versehen ist, wobei der Hebelmechanismus (16) mit dem ersten und zweiten Trennelement (12,14) verbunden ist und bei der Betätigung diese relativ zueinander bewegt, so dass ein Trennschnitt ausgeführt wird, und wobei der Hebelmechanismus (16) zusammen mit den Trennelementen (12,14) eine sich derart verändernde Kraftübersetzung aufweist, dass bei der Betätigung des Hebelmechanismus (16) die Schneidkraft der Trennelemente (12, 24) ansteigt,
**dadurch gekennzeichnet, dass**
die Schneidposition der Schneidkanten (12b, 14b) der Trennelemente (12,14) erst erreicht ist, wenn zumindest 50% des Hebelwegs des Hebelmechanismus (16) zurückgelegt sind.

2. Stabtrennzange (10) nach Anspruch 1, bei der zumindest ein Trennelement (12,14) abgeknickt ausgebildet ist.

3. Stabtrennzange (10) nach einem der vorhergehenden Ansprüche, bei der der Hebelmechanismus (16) zusammen mit den Trennelementen (12,14) zumindest einen Kniehebel ausbildet.

4. Stabtrennzange (10) nach einem der vorhergehenden Ansprüche, bei der der Hebelmechanismus (16) eine Schnittstelle (27) für das Befestigen von Handhebeln (22, 23) und/oder von Hebelverlängerungen aufweist.

5. Stabtrennzange (10) nach einem der vorhergehenden Ansprüche, bei der zumindest eine Aufnahme (12a, 14a) des ersten und zweiten Trennelements (12,14) eine Aussparung aufweist, die länglich ausgebildet ist.

6. Stabtrennzange (10) nach Anspruch 5, bei der die Aussparung in beiden Trennelementen (12,14) ausgebildet ist.

7. Stabtrennzange (10) nach einem der vorhergehenden Ansprüche, bei der die Aufnahmen (12a, 14a) der Trennelemente (12,14) bei betätigtem Hebelmechanismus (16) in der Endstellung nicht komplett abgedeckt sind.

8. Stabtrennzange (10) nach einem der vorhergehenden Ansprüche, bei der eine Mehrzahl einander entsprechenden Aufnahmen (12a, 14a; 9) an den Trennelementen vorgesehen sind.

9. Stabtrennzange (10) nach einem der vorhergehenden Ansprüche, bei der die Trennelemente (12,14) an einem gemeinsamen Gelenk (20) verbunden sind und sich bei Betätigung des Hebelmechanismus (16) rotatorisch um dieses Gelenk (20) bewegen.

10. Stabtrennzange (10) nach einem der vorhergehenden Ansprüche, bei der die Schneidposition der Schneidkanten (12b, 14b) der Trennelemente (12,14) erst erreicht ist, wenn die miteinander verbundenen Schenkel (13,15) des Hebelmechanismus zueinander in einem Winkel von 0° bis 45° Grad oder 135° bis 180° Grad stehen
